# EUROPÄISCHE PATENTSCHRIFT

(11) **EP 1 239 827 B1**
(45) Veröffentlichungstag und Bekanntmachung des Hinweises auf die Patenterteilung: **13.10.2004**
(21) Anmeldenummer: 00981379.1
(22) Anmeldetag: 15.12.2000
(51) Int. Cl.: A61K 7/50, C11D 1/645, C11D 1/835, C11D 1/62, C11D 1/90

(54) **TRANSPARENTE AVIVAGEMITTEL**
TRANSPARENT SOFTENING AGENTS
AGENTS D'AVIVAGE TRANSPARENTS

(30) Priorität: 24.12.1999 DE 19962874
(43) Veröffentlichungstag der Anmeldung: 18.09.2002
(73) Patentinhaber: Cognis Iberia, S.L., 08755 Castellbisbal, (Barcelona) (ES)
(72) Erfinder: BIGORRA LLOSAS, Joaquin, 08203 Sabadell (ES); BONASTRE GILABERT, Nuria, 08210 Barbera del Vall (ES); PI SUBIRANA, Rafael, 08400 Granollers (ES); CALDERO, Gabriela, 08034 Barcelona (ES)
(74) Vertreter: Fabry, Bernd, Dr.
(86) Internationale Anmeldenummer: PCT/EP2000/012811
(87) Internationale Veröffentlichungsnummer: WO 2001/047489

(56) Entgegenhaltungen:
- EP-A- 0 786 250
- WO-A-99/18178
- DE-A- 2 500 241
- DE-A- 3 032 216
- DE-A- 19 751 151
- DE-C- 19 715 835
- US-A- 5 880 299

## Beschreibung

### Gebiet der Erfindung

Die Erfindung betrifft transparente Avivagemittel aus speziellen Esterquats und Hilfsstoffen, wie Fettsäureamidoamine bzw. deren Quaternierungsprodukte, Betaine, nichtionische Tenside, Polyole und/oder deren Derivate, Alkohole und/oder Hydrotrope.

### Stand der Technik

Unter der Bezeichnung "Esterquats" werden im allgemeinen quaternierte Fettsäuretriethanolaminestersalze verstanden, die sich in breitem Umfang sowohl für die Faser- als auch für die Haaravivage eignen. In den vergangenen Jahren haben diese Stoffe infolge ihrer besseren ökotoxikologischen Verträglichkeit konventionelle quartäre Ammoniumverbindungen wie z.B. das bekannte Distearyldimethylammoniumchlorid zu einem guten Teil vom Markt verdrängt. Übersichten zu diesem Thema sind beispielsweise von O. Ponsati in **C.R. CED-Kongress, Barcelona, 1992, S.167,** R. Puchta et al. in **Tens.Surf.Det., 30, 186 (1993),** M.Brock in **Tens. Surf. Det. 30, 394 (1993)** und R. Lagerman et al, in **J.Am.Oil.Chem.Soc., 71, 97 (1994)** erschienen. Des weiteren sei auf die Druckschriften **US 5,880,299, DE-A1 19751151, WO 99/18178** und **DE-C1 19715835** verwiesen, die ebenfalls Reinigungsmittel mit einem Gehalt an Esterquats und verschiedenen Hilfsstoffen beschreiben.

Obwohl Esterquats des Stands der Technik über sehr gute anwendungstechnische Eigenschaften verfügen sowie eine zufriedenstellende biologische Abbaubarkeit und eine gute hautkosmetische Verträglichkeit besitzen, sind die aus dem Stand der Technik bekannten Zubereitungen mit Esterquats trüb.

Demzufolge hat die Aufgabe der Erfindung darin bestanden, Zubereitungen mit Esterquats zur Verfügung zu stellen, welche auch nach Lagerung nicht austrüben und gleichzeitig ein gutes Avivage- und Antistatikverhalten aufweisen. Darüber hinaus sollen derartige Zubereitungen das Bügeln von Textilien erleichtern.

### Beschreibung der Erfindung

Gegenstand der Erfindung sind transparente Avivagemittel, enthaltend
(a) Esterquats, dadurch erhältlich, dass man Alkanolamine mit einer Mischung aus Fettsäuren und Dicarbonsäuren umsetzt, die resultierenden Ester gegebenenfalls alkoxyliert und anschließend in an sich bekannter Weise quaterniert und
(b) quaternierte Fettsäureamidoamine.

Überraschenderweise wurde gefunden, dass man durch Abmischung spezieller Esterquats mit Fettsäureamidoaminen bzw. deren Quaternierungsprodukten transparente Zubereitungen erhält, welche auch nach Lagerung nicht trüb werden und weiterhin ausgezeichnete Haar- und Faseravivageeigenschaften aufzeigen. Darüber hinaus lassen sich Fasern, welche mit diesen Zubereitungen behandelt wurden, wesentlich leichter bügeln.

### Esterquats

Alkanolamine, die im Sinne der Erfindung als zentrale Stickstoffverbindungen in Betracht kommen, können einen Alkanrest mit 1 bis 4 Kohlenstoffatomen enthalten. Vorzugsweise werden Triethanolamin sowie deren Anlagerungsprodukte von 1 bis 10 und vorzugsweise 2 bis 5 Mol Ethylenoxid eingesetzt. Weiterhin kommen Mischungen von Triethanolaminen in Frage, wie beispielsweise Methyldiethanolamin mit Triethanolamin und dergleichen. Unter Fettsäuren sind aliphatische Carbonsäuren der **Formel (I)** zu verstehen,

**R**^{**1**}**CO-OH** (I)

in der R¹CO für einen aliphatischen, linearen oder verzweigten Acylrest mit 6 bis 22 Kohlenstoffatomen und 0 und/oder 1, 2 oder 3 Doppelbindungen steht. Typische Beispiele sind Capronsäure, Caprylsäure, 2-Ethylhexansäure, Caprinsäure, Laurinsäure, Isotridecansäure, Myristinsäure, Palmitinsäure, Palmoleinsäure, Stearinsäure, Isostearinsäure, Ölsäure, Elaidinsäure, Petroselinsäure, Linolsäure, Linolensäure, Elaeostearinsäure, Arachinsäure, Gadoleinsäure, Behensäure und Erucasäure sowie deren technische Mischungen, die z.B. bei der Druckspaltung von natürlichen Fetten und Ölen, bei der Reduktion von Aldehyden aus der Roelen'schen Oxosynthese oder der Dimerisierung von ungesättigten Fettsäuren anfallen. Bevorzugt sind technische Fettsäuren mit 12 bis 18 Kohlenstoffatomen wie beispielsweise Kokos-, Palm-, Palmkern- oder Talgfettsäure, vorzugsweise in gehärteter bzw. teilgehärteter Form.

Dicarbonsäuren, die im Sinne der Erfindung als Einsatzstoffe in Betracht kommen, folgen der Formel **(II)**,

**HOOC-[X]-COOH (II)**

in der X für eine gegebenenfalls hydroxysubstituierte Alkylengruppe mit 1 bis 10 Kohlenstoffatomen steht. Typische Beispiele sind Bemsteinsäure, Maleinsäure, Glutarsäure, 1,12-Dodecandisäure und insbesondere Adipinsäure.

Die Fettsäuren und die Dicarbonsäuren können im molaren Verhältnis von 1 : 10 bis 10 : 1 eingesetzt werden. Es hat sich jedoch als vorteilhaft erwiesen, ein molares Verhältnis von 1 : 1 bis 4 : 1 und insbesondere 1,5 : 1 bis 3 : 1 einzustellen. Die Trialkanolamine einerseits und die Säuren - also Fettsäuren und Dicarbonsäuren zusammengenommen - können im molaren Verhältnis 1 : 1,2 bis 1 : 2,4 eingesetzt werden. Als optimal hat sich ein molares Verhältnis Trialkanolamin : Säuren von 1 : 1,5 bis 1 : 1,8 erwiesen. Die Veresterung kann in an sich bekannter Weise durchgeführt werden, wie sie beispielsweise in der Internationalen Patentanmeldung **WO 91/01295** beschrieben wird. Vorteilhafterweise erfolgt die Veresterung bei Temperaturen von 120 bis 220 und insbesondere 130 bis 170°C und Drücken von 0,01 bis 1 bar. Als geeignete Katalysatoren haben sich hypophosphorige Säuren bzw. deren Alkalisalze, vorzugsweise Natriumhypophosphit bewährt, die in Mengen von 0,01 bis 0,1 und vorzugsweise 0,05 bis 0,07 Gew.-% - bezogen auf die Einsatzstoffe - eingesetzt werden können. Im Hinblick auf eine besonders hohe Farbqualität und -stabilität hat sich die Mitverwendung von Alkaliund/oder Erdalkaliborhydriden, wie beispielsweise Kalium-, Magnesium- und insbesondere Natriumborhydrid als vorteilhaft erwiesen. Die Co-Katalysatoren setzt man üblicherweise in Mengen von 50 bis 1000 und insbesondere 100 bis 500 ppm - wieder bezogen auf die Einsatzstoffe - ein. Entsprechende Verfahren sind auch Gegenstand der beiden Deutschen Patentschriften **DE 4308792 C1** und **DE 4409322 C1,** auf deren Lehren hiermit ausdrücklich Bezug genommen wird. Es ist möglich, Mischungen der Fettsäuren und Dicarbonsäuren einzusetzen oder aber die Veresterung mit den beiden Komponenten nacheinander durchzuführen.

US-A-5 880 299, DE-19751151-A, und DE-19715835-A offenbaren Mittel enthaltend Esterquats erhältlich aus der Unsetzung von Mischungen von Fettsäuren und Dicarbonsäuren mit Alkanolaminen, und anschliessender Quaternisierung. EP-A-0 786 250 und WO-9 918 178-A offenbaren Mittel enthaltend Esterquats und Fettsäureamidoanine.

Zur Herstellung von polyalkylenoxidhaltigen Esterquats kann man nach zwei Alternativen verfahren. Zum einen kann man ethoxylierte Trialkanolamine einsetzen. Dies hat den Vorteil, dass die Alkylenoxiverteilung im später resultierenden Esterquat bezüglich der drei OH-Gruppen des Amins annähernd gleich ist. Nachteilig ist jedoch, dass die Veresterung aus sterischen Gründen schwieriger wird. Die Methode der Wahl besteht daher darin, den Ester vor der Quaternierung zu alkoxylieren. Dies kann in an sich bekannter Weise geschehen, d.h. in Anwesenheit basischer Katalysatoren und bei erhöhten Temperaturen. Als Katalysatoren kommen beispielsweise Alkali- und Erdalkalihydroxide und -alkoholate, vorzugsweise Natriumhydroxid und insbesondere Natriummethanolat in Betracht; die Einsatzmenge liegt üblicherweise bei 0,5 bis 5 und vorzugsweise 1 bis 3 Gew.-% - bezogen auf die Einsatzstoffe. Bei Verwendung dieser Katalysatoren werden in erster Linie freie Hydroxylgruppen alkoxyliert. Setzt man als Katalysatoren jedoch calcinierte oder mit Fettsäuren hydrophobierte Hydrotalcite ein, kommt es auch zu einer Insertion der Alkylenoxide in die Esterbindungen. Diese Methode ist bevorzugt, wenn man eine Alkylenoxidverteilung wünscht, die der bei Einsatz von alkoxylierten Trialkanolaminen nahe kommt. Als Alkylenoxide können Ethylen- und Propylenoxid sowie deren Gemische (Random- oder Blockverteilung) eingesetzt werden. Die Reaktion wird üblicherweise bei Temperaturen im Bereich von 100 bis 180°C durchgeführt. Durch den Einbau von im Durchschnitt 1 bis 10 Mol Alkylenoxid pro Mol Ester wird die Hydrophilie der Esterquats gesteigert, die Löslichkeit verbessert und die Reaktivität gegenüber anionischen Tensiden herabgesetzt.

Die Quaternierung der Fettsäure/Dicarbonsäuretrialkanolaminester kann in an sich bekannter Weise durchgeführt werden. Obschon die Umsetzung mit den Alkylierungsmitteln auch in Abwesenheit von Lösungsmitteln durchgeführt werden kann, empfiehlt sich die Mitverwendung zumindest von geringen Mengen Wasser oder niederen Alkoholen, vorzugsweise Isopropylalkohol, zur Herstellung von Konzentraten, die einen Feststoffanteil von mindestens 80 und insbesondere mindestens 90 Gew.-% aufweisen. Als Alkylierungsmittel kommen Alkylhalogenide wie beispielsweise Methylchlorid, Dialkylsulfate wie beispielsweise Dimethylsulfat oder Diethylsulfat oder Dialkylcarbonate wie beispielsweise Dimethylcarbonat oder Diethylcarbonat in Frage.

Üblicherweise werden die Ester und die Alkylierungsmittel im molaren Verhältnis 1 : 0,95 bis 1 : 1,05, also annähernd stöchiometrisch eingesetzt. Die Reaktionstemperatur liegt gewöhnlich bei 40 bis 80 und insbesondere bei 50 bis 60°C. Im Anschluß an die Reaktion empfiehlt es sich, nichtumgesetztes Alkylierungsmittel durch Zugabe beispielsweise von Ammoniak, einem (Alkanol)amin, einer Aminosäure oder einem Oligopeptid zu zerstören, wie dies beispielsweise in der Deutschen Patentanmeldung **DE 4026184 A1** beschrieben wird. Die erfindungsgemäßen Mittel können die Esterquats in Mengen von 5 bis 70, vorzugsweise 15 bis 60 und insbesondere 30 bis 40 Gew.-% - bezogen auf die Endkonzentration - enthalten.

### Quaternierte Fettsäureamidoamine

Quaternierte Fettsäureamidoamine, die als Komponente (b) in Frage kommen, stellen Kondensationsprodukte von Fettsäuren mit gegebenenfalls ethoxylierten Di- oder Oligoaminen dar, die anschließend einer Alkylierung unterworfen werden und vorzugsweise der Formel (III) folgen, 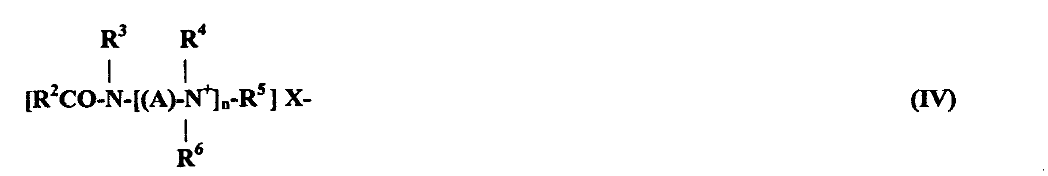 in der R²CO für einen linearen oder verzweigten, gesättigten oder ungesättigten Acylrest mit 6 bis 22 Kohlenstoffatomen, R³ für Wasserstoff oder einen gegebenenfalls hydroxysubstituierten Alkylrest mit 1 bis 4 Kohlenstoffatomen, R⁴ für Wasserstoff, eine (CH₂CH₂O)ₘH-Gruppe oder einen gegebenenfalls hydroxysubstituierten Alkylrest mit 1 bis 4 Kohlenstoffatomen, R⁵ für R²CO, Wasserstoff, eine (CH₂CH₂O)ₘH-Gruppe oder einen gegebenenfalls hydroxysubstituierten Alkylrest mit 1 bis 4 Kohlenstoffatomen, R⁶ für einen Alkylrest mit 1 bis 4 Kohlenstoffatomen, A für eine lineare oder verzweigte Alkylengruppe mit 1 bis 6 Kohlenstoffatomen, n für Zahlen von 1 bis 4, m für Zahlen von 1 bis 30 und X für Halogenid, speziell Chlorid, oder Alkylsulfat, vorzugsweise Methylsulfat steht. Typische Beispiele sind quaternierte Kondensationsprodukte von Capronsäure, Caprylsäure, 2-Ethylhexansäure, Caprinsäure, Laurinsäure, Isotridecansäure, Myristinsäure, Palmitinsäure, Palmoleinsäure, Stearinsäure, Isostearinsäure, Ölsäure, Elaidinsäure, Petroselinsäure, Linolsäure, Linolensäure, Elaeostearinsäure, Arachinsäure, Gadoleinsäure, Behensäure und Erucasäure sowie deren technische Mischungen mit Ethylendiamin, Propylendiamin, Diethylentriamin, Dipropylentriamin, Triethylentetramin, Tripropylentetramin sowie deren Addukten mit 1 bis 30, vorzugsweise 5 bis 15 und insbesondere 8 bis 12 Mol Ethylenoxid. Der Einsatz von ethoxylierten Fettsäureamidoaminen ist dabei bevorzugt, weil sich auf diese Weise die Hydrophilie der Emulgatoren exakt auf die zu emulgierenden Wirkstoffe einstellen lässt. Die Quaternierungsprodukte können hergestellt werden, indem man die Amidoamine mit geeigneten Alkylierungsmitteln, wie beispielsweise Methylchlorid oder insbesondere Dimethylsulfat nach an sich bekannten Verfahren umsetzt. Es können des weiteren auch Mischungen von Fettsäureamidoaminen und deren Quaternierungsprodukten eingesetzt werden, welche man besonders einfach herstellt, in dem man die Quaternierung nicht vollständig, sondern nur bis zu einem gewünschten Grad durchführt. Die erfindungsgemäßen Mittel können die quaternierten Fettsäureamidoamine in Mengen von 0,1 bis 50, vorzugsweise 1 bis 30 und insbesondere 2 bis 10 Gew.-% bezogen auf die Endkonzentration - enthalten.

### Gewerbliche Anwendbarkeit

In einer bevorzugten Ausführungsfbrm der Erfindung enthalten die transparenten Avivagemittel - bezogen auf die Endkonzentration -
(a) 5 bis 90, vorzugsweise 15 bis 80 und insbesondere 30 bis 40 Gew.-% Esterquats,
(b) 0,1 bis 50, vorzugsweise 1 bis 30 und insbesondere 2 bis 10 Gew.-% quaternierte Fettsäureamidoamine
mit der Maßgabe, dass sich die Mengenangaben mit Wasser und gegebenenfalls weiteren Hilfs- und Zusatzstoffen zu 100 Gew.-% ergänzen.

Die erfindungsgemäßen Avivagemittel können zur Herstellung von kosmetischen und/oder pharmazeutischen Reinigungsmitteln zur Haaravivage, wie beispielsweise Cremes, Gele, Lotionen, Emulsionen, Wachs/Fett-Massen, oder Salben sowie zur Herstellung von Reinigungsmitteln, vorzugsweise Waschmitteln, Wäscheweichspülern und dergleichen eingesetzt werden. Sie können ferner als weitere Zusatzstoffe milde Tenside, Ölkomponenten, Emulgatoren, Überfettungsmittel, Perlglanzwachse, Konsistenzgeber, Verdickungsmittel, Polymere, Siliconverbindungen, Fette, Wachse, Stabilisatoren, biogene Wirkstoffe, Deowirkstoffe, Antischuppenmittel, Filmbildner, Quellmittel, weitere UV-Lichtschutzfaktoren, Antioxidantien, Hydrotrope, Konservierungsmittel, Insektenrepellentien, Selbstbräuner, Solubilisatoren, Parfümöle, Farbstoffe, keimhemmende Mittel und dergleichen enthalten.

Typische Beispiele für geeignete milde, d.h. besonders hautverträgliche **Tenside** sind Fettalkoholpolyglycolethersulfate, Monoglyceridsulfate, Mono- und/oder Dialkylsulfosuccinate, Fettsäureisethionate, Fettsäuresarcosinate, Fettsäuretauride, Fettsäureglutamate, α-Olefinsulfonate, Ethercarbonsäuren, Alkyloligoglucoside, Fettsäureglucamide, Alkylamidobetaine und/oder Proteinfettsäurekondensate, letztere vorzugsweise auf Basis von Weizenproteinen.

Als **Ölkomponenten** der erforderlichen Polarität kommen insbesondere Guerbetalkohole auf Basis von Fettalkoholen mit 6 bis 18, vorzugsweise 8 bis 10 Kohlenstoffatomen, Ester von verzweigten C₆-C₁₃-Carbonsäuren mit linearen C₆-C₂₂-Fettalkoholen, Ester von linearen C₆-C₂₂-Fettsäuren mit verzweigten Alkoholen, insbesondere 2-Ethylhexanol, Ester von Hydroxycarbonsäuren mit linearen oder verzweigten C₆-C₂₂-Fettalkoholen, insbesondere Dioctyl Malate, Ester von linearen und/oder verzweigten Fettsäuren mit mehrwertigen Alkoholen (wie z.B. Propylenglycol, Dimerdiol oder Trimertriol) und/oder Guerbetalkoholen, flüssige Mono-/Di-/Triglyceridmischungen auf Basis von C₆-C₁₈-Fettsäuren, Ester von C₆-C₂₂-Fettalkoholen und/oder Guerbetalkoholen mit aromatischen Carbonsäuren, insbesondere Benzoesäure, Ester von C₂-C₁₂-Dicarbonsäuren mit linearen oder verzweigten Alkoholen mit 1 bis 22 Kohlenstoffatomen oder Polyolen mit 2 bis 10 Kohlenstoffatomen und 2 bis 6 Hydroxylgruppen, verzweigte primäre Alkohole, substituierte Cyclohexane, lineare und verzweigte C₆-C₂₂-Fettalkoholcarbonate, Guerbetcarbonate, Ester der Benzoesäure mit linearen und/oder verzweigten C₆-C₂₂-Alkoholen (z.B. Finsolv® TN), lineare oder verzweigte, symmetrische oder unsymmetrische Dialkylether mit 6 bis 22 Kohlenstoffatomen pro Alkylgruppe, Ringöffnungsprodukte von epoxidierten Fettsäureestern mit Polyolen in Betracht.

Als **Überfettungsmittel** können Substanzen wie beispielsweise Lanolin und Lecithin sowie polyethoxylierte oder acylierte Lanolin- und Lecithinderivate, Polyolfettsäureester, Monoglyceride und Fettsäurealkanolamide verwendet werden, wobei die letzteren gleichzeitig als Schaumstabilisatoren dienen.

Als **Perlglanzwachse** kommen beispielsweise in Frage: Alkylenglycolester, speziell Ethylenglycoldistearat; Fettsäurealkanolamide, speziell Kokosfettsäurediethanolamid; Partialglyceride, speziell Stearinsäuremonoglycerid; Ester von mehrwertigen, gegebenenfalls hydroxysubstituierte Carbonsäuren mit Fettalkoholen mit 6 bis 22 Kohlenstoffatomen, speziell langkettige Ester der Weinsäure; Fettstoffe, wie beispielsweise Fettalkohole, Fettketone, Fettaldehyde, Fettether und Fettcarbonate, die in Summe mindestens 24 Kohlenstoffatome aufweisen, speziell Lauron und Distearylether; Fettsäuren wie Stearinsäure, Hydroxystearinsäure oder Behensäure, Ringöffnungsprodukte von Olefinepoxiden mit 12 bis 22 Kohlenstoffatomen mit Fettalkoholen mit 12 bis 22 Kohlenstoffatomen und/oder Polyolen mit 2 bis 15 Kohlenstoffatomen und 2 bis 10 Hydroxylgruppen sowie deren Mischungen.

Als **Konsistenzgeber** kommen in erster Linie Fettalkohole oder Hydroxyfettalkohole mit 12 bis 22 und vorzugsweise 16 bis 18 Kohlenstoffatomen und daneben Partialglyceride, Fettsäuren oder Hydroxyfettsäuren in Betracht. Bevorzugt ist eine Kombination dieser Stoffe mit Alkyloligoglucosiden und/oder Fettsäure-N-methylglucamiden gleicher Kettenlänge und/oder Polyglycerinpoly-12-hydroxystearaten. Geeignete **Verdickungsmittel** sind beispielsweise Aerosil-Typen (hydrophile Kieselsäuren), Polysaccharide, insbesondere Xanthan-Gum, Guar-Guar, Agar-Agar, Alginate und Tylosen, Carboxymethylcellulose und Hydroxyethylcellulose, ferner höhermolekulare Polyethylenglycolmono- und -diester von Fettsäuren, Polyacrylate, (z.B. Carbopole® von Goodrich oder Synthalene® von Sigma), Polyacrylamide, Polyvinylalkohol und Polyvinylpyrrolidon, Tenside wie beispielsweise ethoxylierte Fettsäureglyceride, Ester von Fettsäuren mit Polyolen wie beispielsweise Pentaerythrit oder Trimethylolpropan, Fettalkoholethoxylate mit eingeengter Homologenverteilung oder Alkyloligoglucoside sowie Elektrolyte wie Kochsalz und Ammoniumchlorid.

Geeignete **kationische Polymere** sind beispielsweise kationische Cellulosederivate, wie z.B. eine quaternierte Hydroxyethylcellulose, die unter der Bezeichnung Polymer JR 400® von Amerchol erhältlich ist, kationische Stärke, Copolymere von Diallylammoniumsalzen und Acrylamiden, quaternierte Vinylpyrrolidon/Vinylimidazol-Polymere, wie z.B. Luviquat® (BASF), Kondensationsprodukte von Polyglycolen und Aminen, quaternierte Kollagenpolypeptide, wie beispielsweise Lauryldimonium hydroxypropyl hydrolyzed collagen (Lamequat®L/Grünau), quaternierte Weizenpolypeptide, Polyethylenimin, kationische Siliconpolymere, wie z.B. Amidomethicone, Copolymere der Adipinsäure und Dimethylaminohydroxypropyldiethylentriamin (Cartaretine®/Sandoz), Copolymere der Acrylsäure mit Dimethyldiallylammoniumchlorid (Merquat® 550/Chemviron), Polyaminopolyamide, wie z.B. beschrieben in der **FR 2252840 A** sowie deren vernetzte wasserlöslichen Polymere, kationische Chitinderivate wie beispielsweise quaterniertes Chitosan, gegebenenfalls mikrokristallin verteilt, Kondensationsprodukte aus Dihalogenalkylen, wie z.B. Dibrombutan mit Bisdialkylaminen, wie z.B. Bis-Dimethylamino-1,3-propan, kationischer Guar-Gum, wie z.B. Jaguar® CBS, Jaguar® C-17, Jaguar® C-16 der Firma Celanese, quaternierte Ammoniumsalz-Palymere, wie z.B. Mirapol® A-15, Mirapol® AD-1, Mirapol® AZ-1 der Firma Miranol.

Als **anionische, zwitterionische, amphotere und nichtionische Polymere** kommen beispielsweise Vinylacetat/Crotonsäure-Copolymere, Vinylpyrrolidon/Vinylacrylat-Copolymere, Vinylacetat/Butylmaleat/ Isobornylacrylat-Copolymere, Methylvinylether/Maleinsäureanhydrid-Copolymere und deren Ester, unvernetzte und mit Polyolen vernetzte Polyacrylsäuren, Acrylamidopropyltrimethylammoniumchlorid/ Acrylat-Copolymere, Octylacrylamid/Methylmethacrylat/tert.Butylaminoethylmethacrylat/2-Hydroxyproylmethacrylat-Copolymere, Polyvinylpyrrolidon, Vinylpyrrolidon/Vinylacetat-Copolymere, Vinylpyrrolidon/Dimethylaminoethylmethacrylat/Vinylcaprolactam-Terpolymere sowie gegebenenfalls derivatisierte Celluloseether und Silicone in Frage.

Geeignete **Siliconverbindungen** sind beispielsweise Dimethylpolysiloxane, Methylphenylpolysiloxane, cyclische Silicone sowie amino-, fettsäure-, alkohol-, polyether-, epoxy-, fluor-, glykosid- und/oder alkylmodifizierte Siliconverbindungen, die bei Raumtemperatur sowohl flüssig als auch harzförmig vorliegen können. Weiterhin geeignet sind Simethicone, bei denen es sich um Mischungen aus Dimethiconen mit einer durchschnittlichen Kettenlänge von 200 bis 300 Dimethylsiloxan-Einheiten und hydrierten Silicaten handelt. Eine detaillierte Übersicht über geeignete flüchtige Silicone findet sich zudem von Todd et al. in **Cosm.Toil. 91, 27 (1976).**

Typische Beispiele für **Fette** sind Glyceride, als **Wachse** kommen u.a. Bienenwachs, Camaubawachs, Candelillawachs, Montanwachs, Paraffinwachs, hydriertes Ricinusöle, bei Raumtemperatur feste Fettsäureester oder Mikrowachse gegebenenfalls in Kombination mit hydrophilen Wachsen, z.B. Cetylstearylalkohol oder Partialglyceriden in Frage. Als **Stabilisatoren** können Metallsalze von Fettsäuren, wie z.B. Magnesium-, Aluminium- und/oder Zinkstearat bzw. -ricinoleat eingesetzt werden.

Unter **biogenen Wirkstoffen** sind beispielsweise Tocopherol, Tocopherolacetat, Tocopherolpalmitat, Ascorbinsäure, Desoxyribonucleinsäure, Retinol, Bisabolol, Allantoin, Phytantriol, Panthenol, AHA-Säuren, Aminosäuren, Ceramide, Pseudoceramide, essentielle Öle, Pflanzenextrakte und Vitaminkomplexe zu verstehen.

Als **Deowirkstoffe** kommen z.B. Antiperspirantien wie etwa Aluminiumchlorhydate in Frage. Hierbei handelt es sich um farblose, hygroskopische Kristalle, die an der Luft leicht zerfließen und beim Eindampfen wässriger Aluminiumchloridlösungen anfallen. Aluminiumchlorhydrat wird zur Herstellung von schweißhemmenden und desodorierenden Zubereitungen eingesetzt und wirkt wahrscheinlich über den partiellen Verschluß der Schweißdrüsen durch Eiweißund/oder Polysaccharidfällung [vgl. **J.Soc. Cosm.Chem. 24, 281 (1973)].** Unter der Marke Locron® der Hoechst AG, Frankfurt/FRG, befindet beispielsweise sich ein Aluminiumchlorhydrat im Handel, das der Formel [Al₂(OH)₅Cl]*2,5 H₂O entspricht und dessen Einsatz besonders bevorzugt ist [vgl. **J.Pharm.Pharmacol. 26, 531 (1975)].** Neben den Chlorhydraten können auch Aluminiumhydroxylactate sowie saure Aluminium/Zirkoniumsalze eingesetzt werden. Als weitere Deowirkstoffe können Esteraseinhibitoren zugesetzt werden. Hierbei handelt es sich vorzugsweise um Trialkylcitrate wie Trimethylcitrat, Tripropylcitrat, Triisopropylcitrat, Tributylcitrat und insbesondere Triethylcitrat (Hydagen® CAT, Henkel KGaA, Düsseldorf/FRG). Die Stoffe inhibieren die Enzymaktivität und reduzieren dadurch die Geruchsbildung. Wahrscheinlich wird dabei durch die Spaltung des Citronensäureesters die freie Säure freigesetzt, die den pH-Wert auf der Haut soweit absenkt, dass dadurch die Enzyme inhibiert werden. Weitere Stoffe, die als Esteraseinhibitoren in Betracht kommen, sind Sterolsulfate oder -phosphate, wie beispielsweise Lanosterin-, Cholesterin-, Campesterin-, Stigmasterin- undSitosterinsulfat bzw -phosphat, Dicarbonsäuren und deren Ester, wie beispielsweise Glutarsäure, Glutarsäuremonoethylester, Glutarsäurediethylester, Adipinsäure, Adipinsäuremonoethylester, Adipinsäurediethylester, Malonsäure und Malonsäurediethylester, Hydroxycarbnonsäuren und deren Ester wie beispielsweise Citronensäure, Äpfelsäure, Weinsäure oder Weinsäurediethylester. Antibakterielle Wirkstoffe, die die Keimflora beeinflussen und schweißzersetzende Bakterien abtöten bzw. in ihrem Wachstum hemmen, können ebenfalls in den Stiftzubereitungen enthalten sein. Beispiele hierfür sind Chitosan, Phenoxyethanol und Chlorhexidingluconat. Besonders wirkungsvoll hat sich auch 5-Chlor-2-(2,4-dichlorphenoxy)-phenol erwiesen, das unter der Marke Irgasan® von der Ciba-Geigy, Basel/CH vertrieben wird.

Als Antischuppenmittel können Climbazol, Octopirox und Zinkpyrethion eingesetzt werden. Gebräuchliche **Filmbildner** sind beispielsweise Chitosan, mikrokristallines Chitosan, quaterniertes Chitosan, Polyvinylpyrrolidon, Vinylpyrrolidon-Vinylacetat-Copolymerisate, Polymere der Acrylsäurereihe, quaternäre Cellulose-Derivate, Kollagen, Hyaluronsäure bzw. deren Salze und ähnliche Verbindungen.

Als **Quellmittel** für wässrige Phasen können Montmorillonite, Clay Mineralstoffe, Pemulen sowie alkylmodifizierte Carbopoltypen (Goodrich) dienen. Weitere geeignete Polymere bzw. Quellmittel können der Übersicht von R.Lochhead in **Cosm.Toil. 108, 95 (1993)** entnommen werden.

Unter **UV-Lichtschutzfaktoren** sind beispielsweise bei Raumtemperatur flüssig oder kristallin vorliegende organische Substanzen (Lichtschutzfilter) zu verstehen, die in der Lage sind, ultraviolette Strahlen zu absorbieren und die aufgenommene Energie in Form längerwelliger Strahlung, z.B. Wärme wieder abzugeben. UVB-Filter können öllöslich oder wasserlöslich sein. Als öllösliche Substanzen sind z.B. zu nennen:
- 4-Aminobenzoesäurederivate, vorzugsweise 4-(Dimeihylamino)benzoesäure-2-ethylhexylester, 4-(Dimethylamino)benzoesäure-2-octylester und 4-(Dimethylamino)-benzoesäureamylester;
- Ester der Salicylsäure, vorzugsweise Salicylsäure-2-ethylhexylester, Salicylsäure-4-isopropylbenzylester, Salicylsäurehomomenthylester;
- Derivate des Benzophenons, vorzugsweise 2-Hydroxy-4-methoxybenzophenon, 2-Hydroxy-4-methoxy-4'-methylbenzophenon, 2,2'-Dihydroxy-4-methoxybenzophenon;
- Ester der Benzalmalonsäure, vorzugsweise 4-Methoxybenzmalonsäuredi-2-ethylhexylester;
- Triazinderivate, wie z.B. 2,4,6-Trianilino-(p-carbo-2'-ethyl-1'-hexyloxy)-1,3,5-triazin und Octyl Triazon, wie in der **EP 0818450 A1** beschrieben;
- Propan-1,3-dione, wie z.B. 1-(4-tert.Butylphenyl)-3-(4'methoxyphenyl)propan-1,3-dion;
- Ketotricyclo(5.2.1.0)decan-Derivate, wie in der **EP 0694521 B1** beschrieben.

Als wasserlösliche Substanzen kommen in Frage:
- 2-Phenylbenzimidazol-5-sulfonsäure und deren Alkali-, Erdalkali-, Ammonium-, Alkylammonium-, Alkanolammonium- und Glucammoniumsalze;
- Sulfonsäurederivate von Benzophenonen, vorzugsweise 2-Hydroxy-4-methoxybenzophenon-5-sulfonsäure und ihre Salze;
- Sulfonsäurederivate des 3-Benzylidencamphers, wie z.B. 4-(2-Oxo-3-bornylidenmethyl)benzolsulfonsäure und 2-Methyl-5-(2-oxo-3-bomyliden)sulfonsäure und deren Salze.

Neben den beiden vorgenannten Gruppen primärer Lichtschutzstoffe können auch sekundäre Lichtschutzmittel vom Typ der **Antioxidantien** eingesetzt werden, die die photochemische Reaktionskette unterbrechen, welche ausgelöst wird, wenn UV-Strahlung in die Haut eindringt. Typische Beispiele hierfür sind Aminosäuren (z.B. Glycin, Histidin, Tyrosin, Tryptophan) und deren Derivate, Imidazole (z.B. Urocaninsäure) und deren Derivate, Peptide wie D,L-Carnosin, D-Carnosin, L-Carnosin und deren Derivate (z.B. Anserin), Carotinoide, Carotine (z.B. α-Carotin, β-Carotin, Lycopin) und deren Derivate, Chlorogensäure und deren Derivate, Liponsäure und deren Derivate (z.B. Dihydroliponsäure), Aurothioglucose, Propylthiouracil und andere Thiole (z.B. Thioredoxin, Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl-, γ-Linoleyl-, Cholesteryl- und Glycerylester) sowie deren Salze, Dilaurylthiodipropionat, Distearylthiodipropionat, Thiodipropionsäure und deren Derivate (Ester, Ether, Peptide, Lipide, Nukleotide, Nukleoside und Salze) sowie Sulfoximinverbindungen (z.B. Buthioninsulfoximine, Homocysteinsulfoximin, Butioninsulfone, Penta-, Hexa-, Heptathioninsulfoximin) in sehr geringen verträglichen Dosierungen (z.B. pmol bis µmol/kg), ferner (Metall)-Chelatoren (z.B. α-Hydroxyfettsäuren, Palmitinsäure, Phytinsäure, Lactoferrin), α-Hydroxysäuren (z.B. Citronensäure, Milchsäure, Apfelsäure), Huminsäure, Gallensäure, Gallenextrakte, Bilirubin, Biliverdin, EDTA, EGTA und deren Derivate, ungesättigte Fettsäuren und deren Derivate (z.B. γ-Linolensäure, Linolsäure, Ölsäure), Folsäure und deren Derivate, Ubichinon und Ubichinol und deren Derivate, Vitamin C und Derivate (z.B. Ascorbylpalmitat, Mg-Ascorbylphosphat, Ascorbylacetat), Tocopherole und Derivate (z.B. Vitamin-E-acetat), Vitamin A und Derivate (Vitamin-A-palmitat) sowie Koniferylbenzoat des Benzoeharzes, Rutinsäure und deren Derivate, α-Glycosylrutin, Ferulasäure, Furfurylidenglucitol, Carnosin, Butylhydroxytoluol, Butylhydroxyanisol, Nordihydroguajakharzsäure, Nordihydroguajaretsäure, Trihydroxybutyrophenon, Harnsäure und deren Derivate, Mannose und deren Derivate, Superoxid-Dismutase, Zink und dessen Derivate (z.B. ZnO, ZnSO₄) Selen und dessen Derivate (z.B. Selen-Methionin), Stilbene und deren Derivate (z.B. Stilbenoxid, trans-Stilbenoxid) und die erfindungsgemäß geeigneten Derivate (Salze, Ester, Ether, Zucker, Nukleotide, Nukleoside, Peptide und Lipide) dieser genannten Wirkstoffe.

Zur Verbesserung des Fließverhaltens können ferner **Hydrotrope,** wie beispielsweise Ethanol, Isopropylalkohol, oder Polyole eingesetzt werden. Polyole, die hier in Betracht kommen, besitzen vorzugsweise 2 bis 15 Kohlenstoffatome und mindestens zwei Hydroxylgruppen. Die Polyole können noch weitere funktionelle Gruppen, insbesondere Aminogruppen, enthalten bzw. mit Stickstoff modifiziert sein. Typische Beispiele sind
- Glycerin;
- Alkylenglycole, wie beispielsweise Ethylenglycol, Diethylenglycol, Propylenglycol, Butylenglycol, Hexylenglycol sowie Polyethylenglycole mit einem durchschnittlichen Molekulargewicht von 100 bis 1.000 Dalton;
- technische Oligoglyceringemische mit einem Eigenkondensationsgrad von 1,5 bis 10 wie etwa technische Diglyceringemische mit einem Diglyceringehalt von 40 bis 50 Gew.-%;
- Methyolverbindungen, wie insbesondere Trimethylolethan, Trimethylolpropan, Trimethylolbutan, Pentaerythrit und Dipentaerythrit;
- Niedrigalkylglucoside, insbesondere solche mit 1 bis 8 Kohlenstoffen im Alkylrest, wie beispielsweise Methyl- und Butylglucosid;
- Zuckeralkohole mit 5 bis 12 Kohlenstoffatomen, wie beispielsweise Sorbit oder Mannit,
- Zucker mit 5 bis 12 Kohlenstoffatomen, wie beispielsweise Glucose oder Saccharose;
- Aminozucker, wie beispielsweise Glucamin;
- Dialkoholamine, wie Diethanolamin oder 2-Amino-1,3-propandiol.

Als **Konservierungsmittel** eignen sich beispielsweise Phenoxyethanol, Formaldehydlösung, Parabene, Pentandiol oder Sorbinsäure sowie die in Anlage 6, Teil A und B der Kosmetikverordnung aufgeführten weiteren Stoffklassen. Als **Insekten-Repellentien** kommen N,N-Diethyl-m-toluamid, 1,2-Pentandiol oder Insekten-Repellent 3535 in Frage, als **Selbstbräuner** eignet sich Dihydroxyaceton.

Als **Parfümöle** seien genannt Gemische aus natürlichen und synthetischen Riechstoffen. Natürliche Riechstoffe sind Extrakte von Blüten (Lilie, Lavendel, Rosen, Jasmin, Neroli, Ylang-Ylang), Stengeln und Blättern (Geranium, Patchouli, Petitgrain), Früchten (Anis, Koriander, Kümmel, Wacholder), Fruchtschalen (Bergamotte, Zitrone, Orangen), Wurzeln (Macis, Angelica, Sellerie, Kardamon, Costus, Iris, Calmus), Hölzern (Pinien-, Sandel-, Guajak-, Zedern-, Rosenholz), Kräutern und Gräsern (Estragon, Lemongras, Salbei, Thymian), Nadeln und Zweigen (Fichte, Tanne, Kiefer, Latschen), Harzen und Balsamen (Galbanum, Elemi, Benzoe, Myrrhe, Olibanum, Opoponax). Weiterhin kommen tierische Rohstoffe in Frage, wie beispielsweise Zibet und Castoreum. Typische synthetische spielsweise Zibet und Castoreum. Typische synthetische Riechstoffverbindungen sind Produkte vom Typ der Ester, Ether, Aldehyde, Ketone, Alkohole und Kohlenwasserstoffe. Riechstoffverbindungen vom Typ der Ester sind z.B. Benzylacetat, Phenoxyethylisobutyrat, p-tert.-Butylcyclohexylacetat, Linalylacetat, Dimethylbenzylcarbinylacetat, Phenylethylacetat, Linalylbenzoat, Benzylformiat, Ethylmethylphenylglycinat, Allylcyclohexylpropionat, Styrallylpropionat und Benzylsalicylat. Zu den Ethern zählen beispielsweise Benzylethylether, zu den Aldehyden z.B. die linearen Alkanale mit 8 bis 18 Kohlenstoffatomen, Citral, Citronellal, Citronellyloxyacetaldehyd, Cyclamenaldehyd, Hydroxycitronellal, Lilial und Bourgeonal, zu den Ketonen z.B. die Jonone, ∝-Isomethylionon und Methylcedrylketon, zu den Alkoholen Anethol, Citronellol, Eugenol, Isoeugenol, Geraniol, Linalool, Phenylethylalkohol und Terpineol, zu den Kohlenwasserstoffen gehören hauptsächlich die Terpene und Balsame. Bevorzugt werden jedoch Mischungen verschiedener Riechstoffe verwendet, die gemeinsam eine ansprechende Duftnote erzeugen. Auch ätherische Öle geringerer Flüchtigkeit, die meist als Aromakomponenten verwendet werden, eignen sich als Parfümöle, z.B. Salbeiöl, Kamillenöl, Nelkenöl, Melissenöl, Minzenöl, Zimtblätteröl, Lindenblütenöl, Wacholderbeerenöl, Vetiveröl, Olibanöl, Galbanumöl, Labolanumöl und Lavandinöl. Vorzugsweise werden Bergamotteöl, Dihydromyrcenol, Lilial, Lyral, Citronellol, Phenylethylalkohol, α-Hexylzimtaldehyd, Geraniol, Benzylaceton, Cyclamenaldehyd, Linalool, Boisambrene Forte, Ambroxan, Indol, Hedione, Sandelice, Citronenöl, Mandarinenöl, Orangenöl, Allylamylglycolat, Cyclovertal, Lavandinöl, Muskateller Salbeiöl, β-Damascone, Geraniumöl Bourbon, Cyclohexylsalicylat, Vertofix Coeur, Iso-E-Super, Fixolide NP, Evemyl, Iraldein gamma, Phenylessigsäure, Geranylacetat, Benzylacetat, Rosenoxid, Romilllat, Irotyl und Floramat allein oder in Mischungen, eingesetzt.

Als **Farbstoffe** können die für kosmetische Zwecke geeigneten und zugelassenen Substanzen verwendet werden, wie sie beispielsweise in der Publikation **"Kosmetische Färbemittel" der Farbstoffkommission der Deutschen Forschungsgemeinschaft, Verlag Chemie, Weinheim, 1984, S.81-106** zusammengestellt sind. Diese Farbstoffe werden üblicherweise in Konzentrationen von 0,001 bis 0,1 Gew.-%, bezogen auf die gesamte Mischung, eingesetzt.

Typische Beispiele für **keimhemmende Mittel** sind Konservierungsmittel mit spezifischer Wirkung gegen gram-positive Bakterien wie etwa 2,4,4'-Trichlor-2'-hydroxydiphenylether, Chlorhexidin (1,6-Di-(4-chlorphenyl-biguanido)-hexan) oder TCC (3,4,4'-Trichlorcarbanilid). Auch zahlreiche Riechstoffe und etherische Öle weisen antimikrobielle Eigenschaften auf. Typische Beispiele sind die Wirkstoffe Eugenol, Menthol und Thymol in Nelken-, Minz- und Thymianöl. Ein interessantes natürliches Deomittel ist der Terpenalkohol Farnesol (3,7,11-Trimethyl-2,6,10-dodecatrien-1-ol), der im Lindenblütenöl vorhanden ist und einen Maiglöckchengeruch hat. Auch Glycerinmonolaurat hat sich als Bakteriostatikum bewährt. Üblicherweise liegt der Anteil der zusätzlichen keimhemmenden Mittel bei etwa 0,1 bis 2 Gew.-%-bezogen auf den auf den Feststoffanteil der Zubereitungen.

Der Gesamtanteil der Zusatzstoffe kann 1 bis 50, vorzugsweise 5 bis 40 Gew.-% - bezogen auf die Mittel - betragen. Die Herstellung der Mittel kann durch übliche Kalt - oder Heißprozesse erfolgen; vorzugsweise arbeitet man nach der Phaseninversionstemperatur-Methode.

### Beispiele

### Beispiele 1 und 2

In einem Rührreaktor wurden 601 g (2,9 mol) teilgehärtete Kokosfettsäure, 303 g (2,08 mol), Adipinsäure und 1,6 g Hypophosphorsäure vorgelegt und bei vermindertem Druck von 20 mbar auf 70°C erhitzt. Anschließend wurden portionsweise 618 g (4,15 mol) Triethanolamin zugetropft und die Temperatur dabei gleichzeitig bis auf 120°C gesteigert. Nach Beendigung der Zugabe wurde der Ansatz auf 170°C erhitzt, der Druck auf 3 mbar abgesenkt und die Mischung über einen Zeitraum von 2,5 h bei diesen Bedingungen gerührt, bis die Säurezahl auf einen Wert unterhalb von 5 mg KOH/g abgesunken war. Anschließend wurde die Mischung auf 60°C abgekühlt, das Vakuum durch Einleiten von Stickstoff gebrochen und 0,6 g Wasserstoffperoxid in Form einer 30 Gew.-%igen wässrigen Lösung zugegeben. Für die Quaternierung wurden 500 g (1,46 mol) des resultierenden Ester in 120 g Propylenglycol gelöst und über einen Zeitraum von 1 h mit einer solchen Geschwindigkeit mit 175 g (1,39 mol) Dimethylsulfat versetzt, dass die Temperatur nicht über 60 bis 70 °C anstieg. Nach Beendigung der Zugabe ließ man den Ansatz weitere 2,5 h rühren, wobei der Gesamtstickstoffgehalt durch Probenentnahme regelmäßig überprüft wurde. Die Reaktion wurde beendet, nachdem ein konstanter Gesamtstickstoffgehalt erreicht wurde. Es wurde ein Produkt mit einem Feststoffgehalt von 85 Gew.-% erhalten.

Es wurden zwei verschiedene Zubereitungen mit Esterquats und Fettsäureamidoaminen hergestellt. Die Ergebnisse sind in Tabelle 1 zusammengefasst. Die Viskosität wurde nach Brookfield (mPas, 23 °C, Spindel TF, 4 Upm) bestimmt. das Erscheinungsbild wurde nach einer Lagerung von 4 Wochen bei 23 °C betrachtet.

**Tabelle 1:**

| **Transparente Avivagemittel - Mengenangaben in Gew.-% - Wasser ad 100 Gew.-%** | | |
|---|---|---|
| **Zusammensetzung** | **1** | **2** |
| Esterquat gemäß Beispiel 1 | 19 | 14 |
| A | 9 | 15 |
| Ricinusöl + 18 EO | 7 | 7 |
| Glycerin | 0 | 4 |
| ***Erscheinungsbild*** | klar | klar |

| | | |
|---|---|---|
| (A) Kondensationsprodukt von C_{12/18}-Kokosfettsäure mit Dimethylaminopropylamin quaternisiert mit Dimethylsulfat in 85 % Isopropaniol | | |

## Patentansprüche

1. Transparente Avivagemittel, enthaltend
(a) Esterquats, dadurch erhältlich, dass man Alkanolamine mit einer Mischung aus Fettsäuren und Dicarbonsäuren umsetzt, die resultierenden Ester gegebenenfalls alkoxyliert und anschließend in an sich bekannter Weise quaterniert und
(b) quaternierte Fettsäureamidoamine.

2. Mittel nach Anspruch 1, **dadurch gekennzeichnet,** das sie als Komponente (a) Esterquats enthalten, die durch Verwendung von Fettsäuren der Formel **(I)**,
**R**^{**1**}**CO-OH (I)**
in der R¹CO für einen aliphatischen, linearen oder verzweigten Acylrest mit 6 bis 22 Kohlenstoffatomen und 0 und/oder 1, 2 oder 3 Doppelbindungen steht und Dicarbonsäuren der Formel **(II)**,
**HOOC-[X]-COOH (II)**
in der X für eine gegebenenfalls hydroxysubstituierte Alkylengruppe mit 1 bis 10 Kohlenstoffatomen steht, erhältlich sind.

3. Mittel nach mindestens einem der Ansprüche 1 bis 3, **dadurch gekennzeichnet, dass** sie quaternierte Fettsäureamidoamine der Formel **(III)** enthalten, 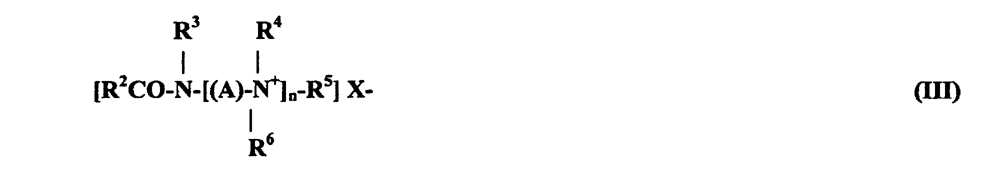 in der R²CO für einen linearen oder verzweigten, gesättigten oder ungesättigten Acylrest mit 6 bis 22 Kohlenstoffatomen, R³ für Wasserstoff oder einen gegebenenfalls hydroxysubstituierten Alkylrest mit 1 bis 4 Kohlenstoffatomen, R⁴ für Wasserstoff, eine (CH₂CH₂O)ₘH-Gruppe oder einen gegebenenfalls hydroxysubstituierten Alkylrest mit 1 bis 4 Kohlenstoffatomen, R⁵ für R²CO, Wasserstoff, eine (CH₂CH₂O)ₘH-Gruppe oder einen gegebenenfalls hydroxysubstituierten Alkylrest mit 1 bis 4 Kohlenstoffatomen, R⁶ für einen Alkylrest mit 1 bis 4 Kohlenstoffatomen, A für eine lineare oder verzweigte Alkylengruppe mit 1 bis 6 Kohlenstoffatomen, n für Zahlen von 1 bis 4, m für Zahlen von 1 bis 30 und X für Halogenid oder Alkylsulfat steht.

4. Mittel nach mindestens einem der Ansprüche 1 bis 4, **dadurch gekennzeichnet, dass** sie - bezogen auf die Endkonzentration -
(a) 5 bis 90 Gew.-% Esterquats und
(b) 0,1 bis 50 Gew.-% quaternierte Fettsäureamidoamine
mit der Maßgabe enthalten, dass sich die Mengenangaben mit Wasser und gegebenenfalls weiteren Zusatzstoffen zu 100 Gew.-% ergänzen.

5. Verwendung von Mitteln nach Anspruch 1 zur Herstellung von Reinigungsmitteln.

## Claims

1. Transparent softeners containing
(a) esterquats obtainable by reacting alkanolamines with a mixture of fatty acids and dicarboxylic acids, optionally alkoxylating the resulting esters and then quaternizing the alkoxylates in known manner and
(b) quaternized fatty acid amidoamines.

2. Softeners as claimed in claim 1, **characterized in that** they contain as component (a) esterquats obtainable by using fatty acids of formula **(I):**
**R**^{**1**}**CO-OH (I)**
in which R¹CO is an aliphatic, linear or branched acyl group containing 6 to 22 carbon atoms and 0 and/or 1, 2 or 3 double bonds, and dicarboxylic acids corresponding to formula **(II)**:
**HOOC-[X]-COOH (II)**
in which X is an optionally hydroxysubstituted alkylene group containing 1 to 10 carbon atoms.

3. Softeners as claimed in at least one of claims 1 to 3, **characterized in that** they contain fatty acid amidoamines corresponding to formula **(III)**: 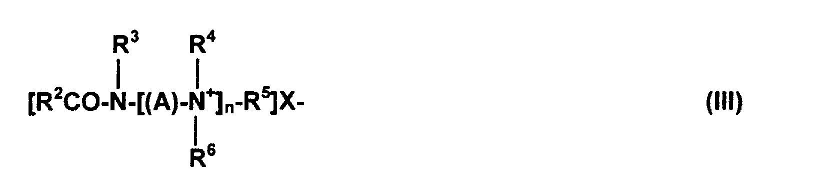 in which R²CO is a linear or branched, saturated or unsaturated acyl group containing 6 to 22 carbon atoms, R³ is hydrogen or an optionally hydroxysubstituted alkyl group containing 1 to 4 carbon atoms, R⁴ is hydrogen, a (CH₂CH₂O)ₘH group or an optionally hydroxysubstituted alkyl group containing 1 to 4 carbon atoms, R⁵ has the same meaning as R²CO or represents hydrogen, a (CH₂CH₂O)ₘH group or an optionally hydroxysubstituted alkyl group containing 1 to 4 carbon atoms, R⁶ is an alkyl group containing 1 to 4 carbon atoms, A is a linear or branched C₁₋₆ alkylene group, n is a number of 1 to 4, m is a number of 1 to 30 and X is halide or alkyl sulfate.

4. Softeners as claimed in at least one of claims 1 to 4, **characterized in that** they contain - based on the final concentration -
(a) 5 to 90% by weight esterquats and
(b) 0.1 to 50% by weight quaternized fatty acid amidoamines,
with the proviso that the quantities mentioned add up to 100% by weight with water and optionally other additives.

5. The use of the softeners claimed in claim 1 for the production of cleaning compositions.

## Revendications

1. Agents d'avivage transparents contenant :
(a) des esters d'ammonium quaternaire, que l'on peut obtenir en faisant réagir des alcanolamines avec un mélange d'acides gras et d'acides dicarboxyliques, en alcoxylant le cas échéant les esters résultants puis en les quaternisant de façon connue, et
(b) des amidoamines d'acides gras quaternisées.

2. Agents selon la revendication 1,
**caractérisés en ce qu'**
ils contiennent comme composant (a) des esters d'ammonium quaternaire que l'on peut obtenir en utilisant des acides gras de formule (I)
R¹CO-OH (I)
dans laquelle R¹CO représente un radical acyle aliphatique, linéaire ou ramifié comportant de 6 à 22 atomes de carbone et 0 et/ou 1, 2 ou 3 doubles liaisons et des acides dicarboxyliques de formule (II)
HOOC-[X]-COOH (II)
dans laquelle X représente un groupe alkylène éventuellement hydroxy-substitué comportant de 1 à 10 atomes de carbone.

3. Agents selon au moins une des revendications là 3,
**caractérisés en ce qu'**
ils contiennent des amidoamines d'acides gras quaternisées de formule (III), 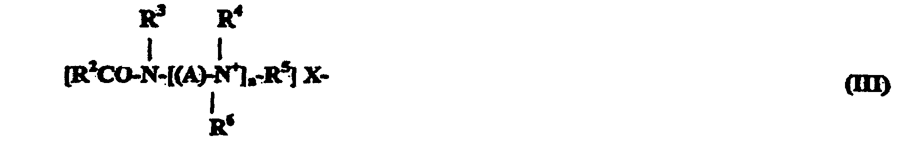 dans laquelle R²CO représente un radical acyle linéaire ou ramifié, saturé ou insaturé comportant de 6 à 22 atomes de carbone, R³ représente un hydrogène ou un radical alkyle éventuellement hydroxy-substitué comportant de 1 à 4 atomes de carbone, R⁴ représente un hydrogène, un groupe (CH₂CH₂O)ₘH ou un radical alkyle éventuellement hydroxy-substitué comportant de 1 à 4 atomes de carbone, R⁵ représente R²CO, un hydrogène, un groupe (CH₂CH₂O)ₘH ou un radical alkyle éventuellement hydroxy-substitué comportant de 1 à 4 atomes de carbone, R⁶ représente un radical alkyle comportant de 1 à 4 atomes de carbone, A représente un groupe alkylène linéaire ou ramifié comportant de 1 à 6 atomes de carbone, n représente des nombres allant de 1 à 4, m représente des nombres allant de 1 à 30 et X représente un halogénure ou un sulfate d'alkyle.

4. Agents selon au moins une des revendications là 4,
**caractérisés en ce qu'**
ils contiennent - par rapport à la concentration finale -
(a) 5 à 90 % en poids d'esters d'ammonium quaternaire et
(b) 0,1 à 50 % en poids d'amidoamines d'acides gras quaternisées,
étant précisé que les indications quantitatives se complètent à 100 % en poids avec de l'eau et le cas échéant d'autres additifs.

5. Utilisation d'agents selon la revendication 1, pour la fabrication de produits de nettoyage.
